Europäisches Patentamt

(19) European Patent Office

Office européen des brevets

(11) Veröffentlichungsnummer : **0 203 214 B1**

(12) EUROPÄISCHE PATENTSCHRIFT

(45) Veröffentlichungstag der Patentschrift :
02.11.89

(51) Int. Cl.⁴ : **A 61 F 2/80**

(21) Anmeldenummer : **85106563.1**

(22) Anmeldetag : **29.05.85**

(54) Verfahren zur Herstellung eines Innentrichters zur Aufnahme einer amputierten Extremität.

(43) Veröffentlichungstag der Anmeldung :
**03.12.86 Patentblatt 86/49**

(45) Bekanntmachung des Hinweises auf die Patenterteilung : **02.11.89 Patentblatt 89/44**

(84) Benannte Vertragsstaaten :
**BE DE FR GB IT NL SE**

(56) Entgegenhaltungen :
**CH--A-- 252 231**
**DE--C-- 843 590**
**DE--C-- 913 466**
**FR--A-- 739 342**
**GB--A-- 391 634**
**US--A-- 3 991 424**

(73) Patentinhaber : **Otto Bock Orthopädische Industrie Besitz- und Verwaltungs-Kommanditgesellschaft Industriestrasse**
**D-3408 Duderstadt 1 (DE)**

(72) Erfinder : **Wellershaus, Ulf**
**Gerlach-Weg 12**
**D-3408 Duderstadt 1 (DE)**
Erfinder : **Fruzinsky, Otto**
**Mathildenstrasse 1**
**D-3408 Duderstadt 1 (DE)**

(74) Vertreter : **Lins, Edgar, Dipl.-Phys. et al Patentanwälte Gramm + Lins Theodor-Heuss-Strasse 2**
**D-3300 Braunschweig (DE)**

## Beschreibung

Die Erfindung betrifft ein Verfahren zur Herstellung eines mit einer Prothese verbindbaren Innentrichters zur Aufnahme eines Stumpfes einer amputierten Extremität, bei dem für die Innentrichterformung ein unter Einwirkung äußerer Einflüsse schrumpffähiger Kunststoff verwendet wird, bei dem ein wenigstens einseitig offenes Rohr aus diesem Kunststoff mit einem kleineren Durchmesser und einer kleineren Länge als der herzustellende Trichter aufgeweitet und gestreckt wird, so daß er in jeder Richtung eine größere Abmessung als der herzustellende Innentrichter aufweist und bei dem das aufgeweitete und gestreckte Formteil durch geeignete Einflüsse zum Schrumpfen gebracht wird.

Die Herstellung derartiger Innentrichter, die die Stümpfe der amputierten Extremitäten unmittelbar aufnehmen und daher extrem gut an die Form des jeweiligen Stumpfes angepaßt sein müssen, geschieht — unabhängig von dem jeweils verwendeten Material — in völlig einheitlicher Weise. Von dem Stumpf wird ein Negativ-Abdruck, beispielsweise aus Gips, hergestellt. Dieser wird mit einem geeigneten Kunststoff gefüllt, woraus der Positiv-Abdruck des Stumpfes entsteht. Auf diesen Positiv-Abdruck wird das Material des Innentrichters aufgebracht, beispielsweise durch Aufgießen eines geeigneten Schaummaterials oder Tiefziehen einer thermoplastischen Kunststoffplatte. Dieses Verfahren ist ersichtlich sehr zeitaufwendig und kostspielig. Bei frisch Amputierten besteht darüber hinaus die Schwierigkeit, daß der Stumpf noch Veränderungen unterworfen ist, die zu der Notwendigkeit führen, einen neuen Schaft mit einem neuen Innentrichter nach einer gewissen Zeit nach der Amputation herzustellen. Auch die Versorgung dieser Patienten (Interimsversorgung) gestaltet sich relativ zeitaufwendig. Durch die mehrfachen Abformvorgänge läßt sich einen optimale Genauigkeit für die Herstellung des Innentrichters nicht erzielen.

Aus der FR-A-739 342 ist ein Verfahren bekannt, bei dem zunächst von dem Stumpf ein Negativ-Abdruck und anschließend ein Positiv-Modell aus Gips hergestellt wird. Dieses Gipsmodell wird mit einem trichterförmig aufgeweiteten Rohr umgeben, das aus einem Material besteht, das bei Wärmeeinwirkung schrumpft. Die gesamte Anordnung wird in heißes Wasser gelegt, wodurch sich das Plastikmaterial der Form des Gipsmodells anschmiegt. Nach Entfernen des Gipsmodells steht ein Trichter zur Verfügung, dessen Form der Form des bebenden Stumpfes entsprechen soll. Auch dieses Verfahren weist durch die zahlreichen Abformvorgänge die Nachteile auf, daß es relativ zeitaufwendig ist und daß die Genauigkeit beeinträchtigt wird.

Der Erfindung liegt daher die Aufgabe zugrunde, eine genaue Herstellung des Innentrichters zu ermöglichen, die weniger zeitaufwendig ist.

Diese Aufgabe wird erfindungsgemäß bei einem Verfahren der eingangs erwähnten Art dadurch gelöst, daß das aufgeweitete und gestreckte Formteil auf den Stumpf selbst aufgesetzt, und daß das Formteil lediglich lokal der Einwirkung der geeigneten Einflüsse unterworfen wird und dadurch nach und nach zum Schrumpfen gebracht wird, bis der Innentrichter überall am Stumpf sicher anliegt.

Erfindungsgemäß wird der Innentrichter somit unmittelbar am Stumpf der amputierten Extremität hergestellt. Dadurch wird die bisher übliche mehrfache Abformung eingespart, die zu Ungenauigkeiten führte und einen erheblichen Zeitaufwand erforderte. Die Anpassung an den Stumpf erfolgt durch eine vorsichtige und jeweils lokal hervorgerufene Schrumpfung des vorher vergrößerten Kunststoffteils. Diese Schrumpfung kann bei den üblichen schrumpffähigen Kunststoffen durch eine lokale Wärmeeinwirkung hervorgerufen werden, die beispielsweise durch Warmluft mittels eines Föns erzeugbar ist. Die Schrumpfung erfolgt dabei bei Temperaturen, die für den menschlichen Körper bequem erträglich sind. Durch die lokale Erwärmung läßt sich eine sehr genaue Anpassung des Innentrichters an den Stumpf auch unter Mitwirkung des Patienten erzielen, indem dieser jederzeit eine Rückmeldung geben kann, wenn das gewünschte Anliegen an dem Stumpf an der erhitzten Stelle erreicht wird.

Für das Aufweiten und Strecken des Kunststoffrohrs eignet sich besonders ein Blasverfahren, bei dem der Kunststoff erhitzt und auf die Kontur einer Blasform durch Einblasen von Druckluft erweitert wird.

Die Anpassung des Innentrichters an den Stumpf läßt sich somit in einfacher Weise vornehmen, wenn ein vorgefertigtes, entsprechend aufgeweitetes Formteil verwendet wird, das aus einem schrumpffähigen Kunststoff besteht und aus einem zumindest einseitig offenen Rohr mit einem kleineren Durchmesser als der herzustellende Innentrichter auf eine Form vergrößert ist, die in jeder Richtung eine größere Innenabmessung als der herzustellende Innentrichter aufweist. Mit einem derartigen Formteil können endgültige Prothesen wie auch Interimsprothesen in orthopädischen Fachwerkstätten in einfacher Weise unter Zuhilfenahme eines Föns hergestellt werden.

In besonders geschickter Weise läßt sich der Innentrichter an die Gegebenheiten des Stumpfes beim Tragen der Prothese anpassen, wenn das Formteil an seiner offenen Oberkante einen umgebogenen Rand aufweist, mit dem es in einen vorgefertigten, an seine Form angepaßten Sitzring einhängbar ist. Diese Anordnung ist insbesondere für die Bildung einer Interimsprothese geeignet. Der Sitzring wird aus einer Mehrzahl von Größen für die betreffende Stumpfgröße ausgewählt. Für den Sitzring ist das aufgeblasene Formteil angepaßt, so daß das Formteil mit dem Sitzring auf den Stumpf geschoben werden kann. Der Sitzring kann umproblematisch eine Übergangsbefestigung für die Prothese, beispielsweise mit Kniegelenk, aufweisen.

Die Erfindung soll im folgenden anhand eines in der Zeichnung dargestellten Ausführungsbeispiels näher erläutert werden. Es zeigen :

Figur 1 — eine Interimsprothese mit einem erfindungsgemäß hergestellten Innentrichter

Figur 2 — einen Sitzring als Einzelteil

Figur 3 — ein erfindungsgemäßes Formteil als Einzelteil

Figur 4 — eine Blasform zur Herstellung eines erfindungsgemäßen Formteils

Figur 5 — die Anpassung des erfindungsgemäßen Formteils an den im Sitzring gehaltenen Stumpf.

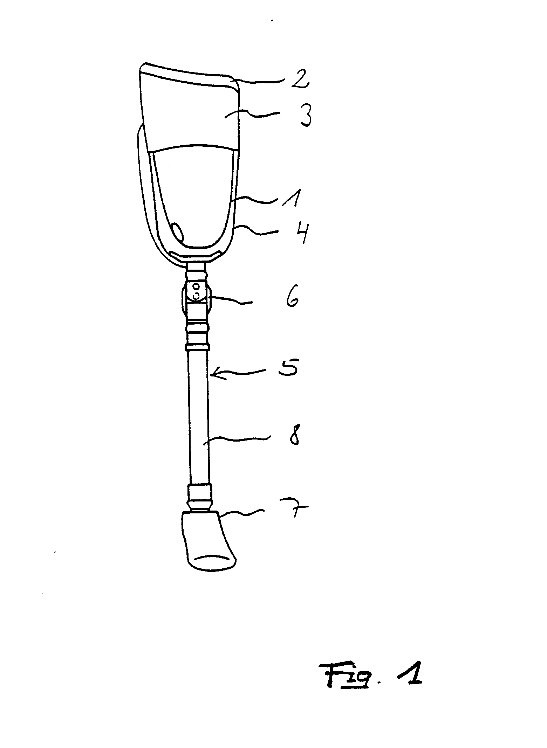

Figur 1 zeigt eine Beinprothese für einen oberschenkelamputierten Patienten. Zur Aufnahme des Oberschenkelstumpfes ist ein Innentrichter 1 vorgesehen, der mit einer am oberen offenen Rand umgebogenen Kante 2 in einen an sich bekannten Sitzring 3 eingehängt ist. Der Sitzring 3 ist mit einem rahmenförmigen Gestell 4 mit einer bekannten Modularprothese 5 verbunden, die ein Kniegelenk und einen künstlichen Fuß aufweist, zwischen denen ein Prothesenrohr 8 verläuft.

Figur 2 zeigt eine Ansicht von schräg hinten auf einen Sitzring 3, der etwas oval geformt ist und einen Oberschenkelstumpf ringförmig hält. Der obere Rand 9 ist nach außen erweitert und weich ausgebildet. Zur Bildung des Innentrichters 1 wird ein oben offenes Formteil 10 in den Sitzring 3 von oben eingehängt. Hierzu weist das Formteil 10 einen an der oberen offenen Kante nach außen abgebogenen Rand 11 auf, der an den Rand 9 des Sitzringes 3 angepaßt ist.

Figur 4 verdeutlicht die Herstellung des Formteils 10 in einer Blasform 12. Die Blasform 12 weist eine Formkontur 13 auf, deren Abmessungen in jeder Richtung größer sind als die Abmessungen des benötigten Innentrichters 1. In die Blasform wird ein Rohling 14 eingehängt, der in Figur 4 gestrichelt eingezeichnet ist. Der Rohling weist die Form eines überdimensionierten Reagenzglases auf. In der Blasform wird der Kunststoff des Rohlings 14 erhitzt und das Innere mit Druckluft beaufschlagt. Dadurch nimmt der Rohling die Kontur 13 der Blasform an, so daß das Formteil 10 gemäß Figur 3 entsteht und nach dem Trennen der beiden Formhälften 15, 16 entnehmbar ist.

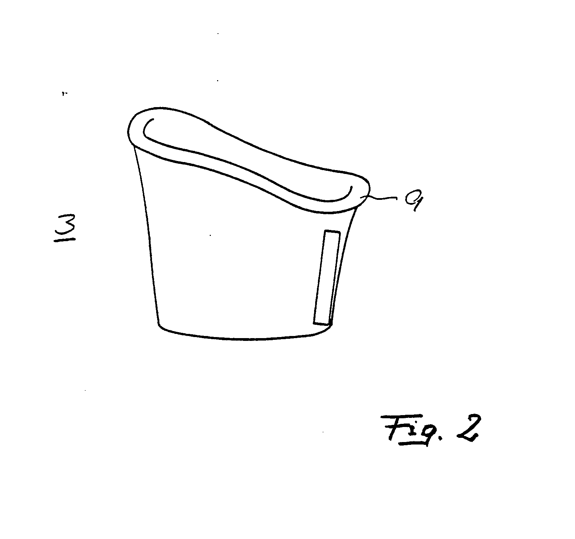

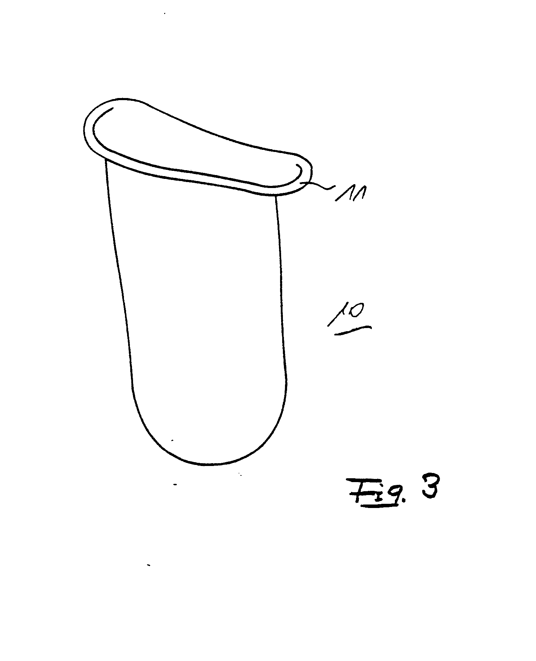

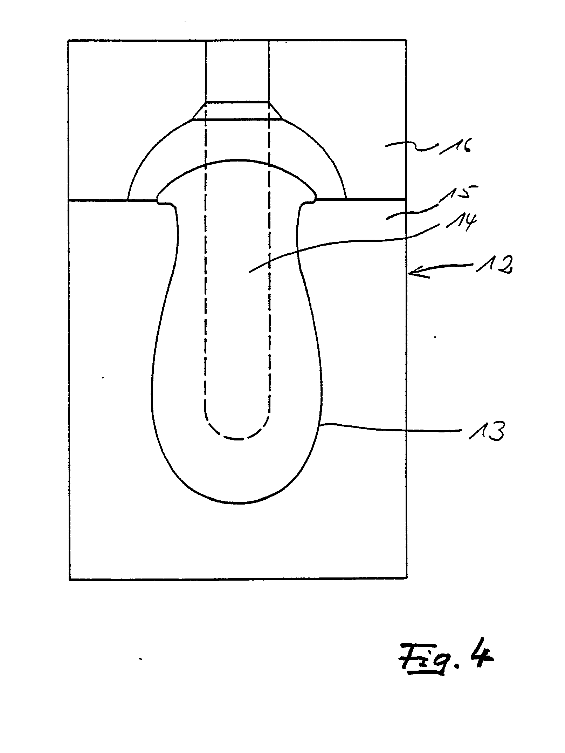

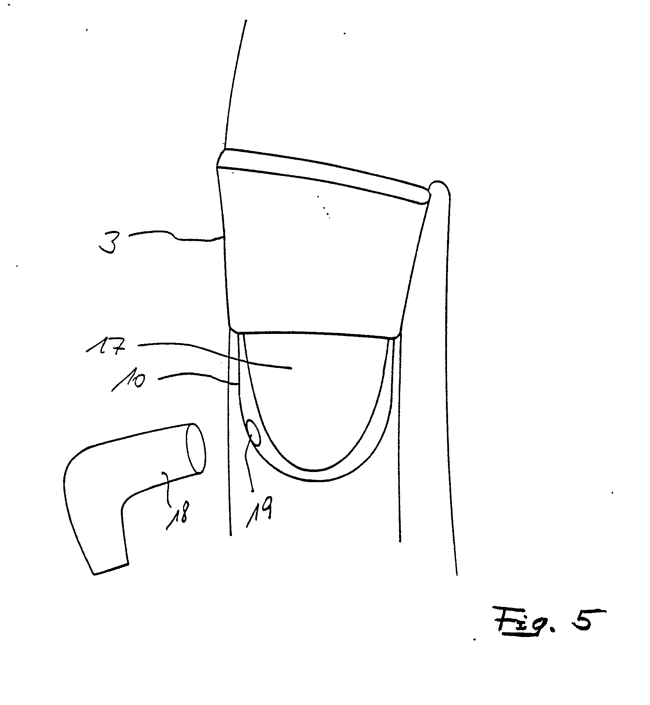

Figur 5 verdeutlicht, daß das Formteil 10 in den Sitzring 3 eingehängt wird und diese Anordnung über einen Stumpf 17 der amputierten Extremität geschoben wird. Das aufgeweitete Formteil 10 weist überall größere Abmessungen auf als der Stumpf 17 bzw. der herzustellende Innentrichter 1, so daß der Stumpf das Formteil 10 nur im oberen Bereich des Sitzringes 3 berührt. Da der Sitzring 3 für den betreffenden Stumpf 17 ausgewählt ist und das Formteil 10 an die Form des Sitzringes 3 angepaßt ist, liegt eine ringförmige Anpassung des Formteils 10 an dem Stumpf 17 im oberen Bereich vor. Die übrige Anpassung erfolgt nun durch Einwirkung von mit einem Fön 18 erzeugten Heißluft auf das Formteil 10, wodurch dieses im Bereich der lokalen Erwärmung schrumpft. Hierdurch kann nach und nach das Formteil 10 an die Form des Stumpfes 17 angepaßt werden, wodurch der an den Stumpf 17 angepaßte Innentrichter 1 entsteht.

Figur 5 läßt noch eine kreisförmige Öffnung 19 des Formteils erkennen, die zur Durchführung des unteren Endes eines Trikotstrumpfes dient, der über den Stumpf 17 gezogen wird, so daß es nicht zu einer Verklebung des Kunststoffes des Formteils 10 mit der Haut des Stumpfes 17 kommt.

In der bisherigen Praxis hat es sich gezeigt, daß nur eine relativ geringe Anzahl von Sitzringen 3 ausreicht, um die verschiedenen Stümpfe 17 zu halten. Demgemäß ist auch nur eine entsprechende Anzahl von vorgefertigten Formteilen 10 erforderlich, um nunmehr für alle Stümpfe 17 den erforderlichen Innentrichter 1 herzustellen. Ist der Innentrichter 1 hergestellt, ist es keine Schwierigkeit, durch Aufbringen weiterer Kunststoffschichten einen fertigen Prothesenschaft zu erzeugen. Dabei können auch geeignete Befestigungsmittel für die restlichen Prothesenelemente vorgesehen werden.

## Patentansprüche

1. Verfahren zur Herstellung eines mit einer Prothese verbindbaren Innentrichters (1) zur Aufnahme eines Stumpfes (17) einer amputierten Extremität, bei dem für die Innentrichterformung ein unter Einwirkung äußerer Einflüsse schrumpffähiger Kunststoff verwendet wird, bei dem ein wenigstens einseitig offenes Rohr (14) aus diesem Kunststoff mit einem kleineren Durchmesser und einer kleineren Länge als der herzustellende Trichter (1) aufgeweitet und gestreckt wird, so daß er in jeder Richtung eine größere Abmessung als der herzustellende Innentrichter (1) aufweist und bei dem das aufgeweitete und gestreckte Formteil (10) durch geeignete Einflüsse zum Schrumpfen gebracht wird, dadurch gekennzeichnet, daß das aufgeweitete und gestreckte Formteil (10) auf den Stumpf (17) selbst aufgesetzt, und daß das Formteil (10) lediglich lokal der Einwirkung der geeigneten Einflüsse unterworfen wird und dadurch nach und nach zum Schrumpfen gebracht wird, bis der Innentrichter (1) überall am Stumpf (17) sicher anliegt.

2. Verfahren nach Anspruch 1, dadurch gekennzeichnet, daß die Erweiterung und Streckung des Rohres (14) in einer Blasform (12) vorgenommen wird, in der der Kunststoff erhitzt und auf die Kontur (13) der Blasform aufgeblasen wird.

3. Verfahren nach Anspruch 1 oder 2, dadurch gekennzeichnet, daß das Schrumpfen des Kunststoffes durch lokale Wärmeeinwirkung hervorgerufen wird.

4. Verfahren nach Anspruch 3, dadurch gekennzeichnet, daß die lokale Wärmeeinwirkung durch Warmluft erzeugt wird.

5. Verfahren nach einem der Ansprüche 1 bis 4, dadurch gekennzeichnet, daß das Formteil (10) mit einem an seiner offenen Oberkante umgebogenen Rand (11) hergestellt wird, mit dem es in einen vorgefertigten, an seine Form angepaßten Sitzring (3) so eingehängt wird, daß der Sitzring (3) das obere Ende des in das Formteil (10) eingeführten Stumpfes (17) hält, während das Formteil (10) nach und nach zum Schrumpfen gebracht wird.

## Claims

1. Method for manufacturing an inner socket (1) connectable to a prothesis for reception of a stump (17) of an amputated extremity, in which there is used for the formation of the inner socket a synthetic material which is shrinkable under the action of an external effect, in which a tube (14) open at at least one end of this synthetic material with a smaller cross-section and a smaller length than the socket (1) to be produced is enlarged and stretched so that it has a larger dimension in each direction than the inner socket (1) to be produced and in which the enlarged and stretched moulded part (10) is caused to shrink by suitable effect, characterized in shat the enlarged and stretched moulded part (10) is fitted on the stump (17) itself and in that the moulded part is subjected to the action of the suitable effect merely locally and thereby gradually caused to shrink, until the inner socket (1) fits the stump (17) securely everywhere.

2. Method according to claim 1, characterized in that the enlarging and stretching of the tube (14) is carried out in a blow mould (12), in which the synthetic material is heated and blown to the contour (13) of the blow mould.

3. Method according to claim 1 or 2, characterized in that the shrinking of the synthetic material is caused by local heat action.

4. Method according to claim 3, characterized in that the local heat action is created by hot air.

5. Method according to one of claims 1 to 4, characterized in that the moulded part (10) is made with a turned over rim (11) at its open upper edge, with which it is so suspended in a prefabricated seating ring (3) adapted to its shape that the seating ring (3) holds the upper end of the stump (17) introduced into the moulded part (10), while the moulded part (10) is gradually caused to shrink.

## Revendications

1. Procédé pour fabriquer une coupelle interne (1) pouvant être reliée à une prothèse et destinée à recevoir le moignon (17) d'une extrémité amputée, dans lequel on utilise, pour le formage de cette coupelle interne, une matière plastique capable de se rétracter sous l'action d'influences extérieures, on fait subir à un tube (14) constitué de cette matière plastique, ouvert au moins d'un côté, et de diamètre et longueur plus petits que la coupelle (1) à fabriquer, un élargissement et un étirement tels qu'il présente en toute direction une dimension supérieure à celle de la coupelle interne (1) à fabriquer, et on amène la pièce de forme (10) élargie et étirée à se rétracter au moyen d'influences appropriées, caractérisé en ce qu'on pose sur le moignon (17) lui-même la pièce de forme élargie et étirée (10), et on ne soumet la pièce de forme (10) que localement à l'action des influences appropriées pour l'amener ainsi à se rétracter progressivement jusqu'à ce que la coupelle interne (1) porte de façon sûre en tout point du moignon (17).

2. Procédé conforme à la revendication 1, caractérisé en ce qu'on élargit et étire le tube (14) dans un moule de soufflage (12), dans lequel la matière plastique est chauffée et soufflée sur le profil (13) du moule de soufflage.

3. Procédé conforme à l'une des revendications 1 ou 2, caractérisé en ce que la rétraction de la matière plastique est provoquée par une action thermique locale.

4. Procédé conforme à la revendication 3, caractérisé en ce qu'on produit l'action thermique locale par de l'air chaud.

5. Procédé conforme à l'une des revendications 1 à 4, caractérisé en ce qu'on fabrique la pièce de forme (10) avec, à son bord supérieur ouvert, une bordure (11) recourbée par laquelle elle peut être suspendue dans un anneau d'appui (3) préfabriqué et adapté à sa forme, de telle sorte que l'anneau d'appui (3) maintient l'extrémité supérieure du moignon (17) introduit dans la pièce de forme (10) tandis qu'on fait rétracter progressivement la pièce de forme (10).

2

3

1

4

6

5

8

7

Fig. 1

3

9

*Fig. 2*

11

10

Fig. 3

Fig. 4

3

17

10

18    19

*Fig. 5*